# EUROPEAN PATENT SPECIFICATION

(11) **EP 1 294 359 B1**
(45) Date of publication and mention of the grant of the patent: **05.04.2006**
(21) Application number: 01943684.9
(22) Date of filing: 29.06.2001
(51) Int. Cl.: A61K 9/16

(54) **PROCESS FOR PREPARING AND HARVESTING CRYSTALLINE PARTICLES**
VERFAHREN ZUR HERSTELLUNG UND SAMMLUNG VON KRISTALLINEN PARTIKELN
PROCEDE DE PREPARATION ET DE COLLECTE DE PARTICULES CRISTALLINES

(30) Priority: 29.06.2000 GB 0015981
(43) Date of publication of application: 26.03.2003
(73) Proprietor: GLAXO GROUP LIMITED, Greenford, Middlesex UB6 ONN (GB)
(72) Inventor: LANCASTER, Robert William, c/o GlaxoSmithKline, Stevenage, Hertfordshire SG1 2NY (GB); SINGH, Hardev, c/o GlaxoSmithKline, Kent DA1 5AH (GB); THEOPHILUS, Andrew Lewis, GlaxoSmithKline, Hertfordshire SG1 2NY (GB)
(74) Representative: Giddings, Peter John
(86) International application number: PCT/GB2001/002922
(87) International publication number: WO 2002/000198

(56) References cited:
- WO-A-00/38811
- WO-A-00/44468
- WO-A-96/32095
- NL-A- 7 501 406

## Description

This invention relates to a novel process for preparing crystalline particles, particularly particles of defined particle size distribution, especially particles of therapeutically useful or carrier substances of a size suitable for inhalation therapy.

Industrial processes for production of many products, particularly pharmaceutical products, require the preparation of pure substances of a defined particle size distribution. Pure substances are frequently prepared by precipitation from solutions of lesser purity. When precipitation takes place relatively slowly (e.g. over a matter of hours), crystals are grown which are frequently of a non-uniform shape and relatively large size.

In the field of inhalation therapy, therapeutic molecules are generally desired of a particle size "suitable for inhalation", which is a term generally taken to indicate an aerodynamic diameter between 1 and 10 µm, especially 1 and 5 µm, particularly 1 and 3 µm. Carrier molecules (such as lactose) for inhaled therapeutic preparations are typically desired of a significantly larger aerodynamic diameter so that they do not penetrate into the upper respiratory tract to the same degree as the active ingredient and an aerodynamic diameter of 100 to 150 µm is generally considered suitable. However this is a generalisation and for some purposes it may well be preferred to use a lower particle size for the carrier, even one comparable to that of the therapeutic substance.

Outside of the inhaled area, modification of the habit and size of crystals is a valuable tool in adjusting and optimising pharmaceutical and biological properties such as flow characteristics, dissolution rate and bioavailability.

Particles of the desired particle size for inhalation therapy are conventionally prepared by milling or micronisation. These processes, depending on the precise conditions adopted, are capable of generating particle distributions which include fractions having particles with the appropriate size. Milling is suitable for preparing particles of the larger size indicated above and micronisation of the smaller size indicated above. However, there are a number of disadvantages associated with milling and micronisation processes including that the fraction having the desired particle size may be relatively small, that there may be generated a significant fraction of particles that are finer than is desired (which may be deleterious e.g. if it affects bioavailability) and that product losses generally may be considerable (e.g. through coating of the machinery). A further property of micronised products is that the surfaces of the particles generated are generally substantially amorphous (i.e. have minimal crystallinity). This may be undesirable when there exists a tendency for the amorphous regions to convert to a more stable crystalline state. Furthermore micronised or milled products may be more susceptible to moisture uptake than crystalline products. Micronisation and milling processes also suffer from the disadvantages that they are relatively energy intensive and require containment and other measures to avoid the risk of dust explosion.

WO 96/32095 describes a process for the preparation of respirable particles comprising dissolving the compound in a solvent, mixing this solution with an anti-solvent by using an ultraturrax and spray-drying the slurry.

International patent application PCT/GB99/04368 (filed but not published before the priority date of this application) describes a process and apparatus for preparing particles which comprises mixing in the presence of ultrasonic radiation a flowing solution of a substance in a liquid solvent with a flowing liquid antisolvent for said substance. International patent application PCT/GB00/04237 describes a process which comprises admitting a stream of a solution of a substance in a liquid solvent and a stream of liquid antisolvent for said substance tangentially into a cylindrical mixing chamber, consequently causing a vortex which results in precipitation of crystalline particles. However, the disadvantage with these 2 processes is that particle growth or agglomeration may occur in the course of isolating the partides from the solvent/anti-solvent mixture. We have now invented an improvement to these processes which is less susceptible to the above mentioned disadvantage.

Thus, according to a first aspect of the invention there is provided a process for preparing crystalline particles of a substance which comprises mixing a flowing Solution of the substance in a liquid solvent with a flowing liquid antisolvent for said substance in order to generate a suspension of crystalline particles in the solvent/anti-solvent the process further comprises the steps of
(a) filtering the suspension of crystalline particles in the solvent/anti-solvent mixture in order to remove the solvent/antisolvent mixture;
(b) washing the filtered particles with water,
(c) resuspending the filtered and washed particles in water:
(d) cooling the resultant suspension of filtered, washed and resuspended particles in wather to freezing point, and
(e) collecting crystalline particles by removal of the water from the cooled suspension by freeze drying.

In a first prefened embodiment of the present invention said mixing comprises mixing in a continuous flow cell in the presence of ultrasonic radiation.

In a second preferred embodiment of the present invention said mixing comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused.

Preferably, the solvent will be miscible with the anti-solvent.

Preferably, the suspension of crystalline particles in the solvent/anti-solvent mixture will be filtered using a wide range of suitable filters known to persons skilled in the art. Examples of filters include sinters (e.g. glass sinters), fibre filters (e.g. paper and nitrocellulose filters) and membrane filters. We have found that a particularly advantageous filtration arrangement involves use of a glass fibre microfilter sandwiched between two Whatman paper filters (e.g. Whatman 54 filters). The particle size of the filter will b e appropriate for the product collected. It is possible to modify the distribution of particles at the fine end by selecting a filter size which allows fines to pass through the filter. Preferably, the filter will be a filter suitable to retain crystalline particles of between 1 and 10µm, most preferably less than 5µm, especially less than 3µm.

It will be appreciated that the anti-solvent (water) used in washing step (b) and resuspension step (c) does not need to be the same anti-solvent that is used in the original process which generates the crystalline particles. Preferably, however, the anti-solvent used in washing step (b) and resuspension step (c) will be the same anti-solvent as is used in the original process.

Preferably, the suspension of crystalline particles obtained in step (d) will be cooled to freezing point using a solid carbon dioxide cooling bath containing a suitable solvent eg. acetone, IMS or methanol.

Where possible, the antisolvent will preferably be water. Preferably, in step (d) the removal of the antisolvent from the cooled suspension is achieved by freeze drying.

The process of the present invention has the advantage of maintaining the original particle diameter of the particles of substance achieved by crystallisation. Conventional collection techniques involve further incubation of the particles in the solvent/antisolvent mixture which may result in undesirable effects such as crystal growth. Wherein the particles are prepared for inhalation therapy, crystal growth is disadvantageous because the particles may grow to a diameter such that they may not be effectively delivered to the lower respiratory airways.

The advantages that the invention may possess include the fact that the process may be performed in a continuous manner without requirements for batch processing, that the process may be scaled up with relative ease and that the process is capable of producing particle size distributions of very high uniformity index.

Surprisingly, the present invention provides processes for removing the solvent from the solvent/antisolvent mixture in order to prevent crystal growth, and as demonstrated in the Examples, also results in particles with more refined particle sizes than achieved with conventional harvesting techniques. Furthermore, when the antisolvent is water, once the solvent has been removed from the solvent/antisolvent mixture (by either procedure) and the mixture is cooled to freezing point, the freeze drying step ensures that the water molecules sublime from the mixture leaving only particles containing the desired substance(s).

The process of the present invention is particularly suitable for preparing particles of substances which are pharmaceutical or carrier substances suitable for inhalation therapy.

Substances suitable for inhalation therapy include substances applied topically to the lung and nose.

Examples of pharmaceutical substances suitable for inhalation therapy include analgesics, e.g., codeine, dihydromorphine, ergotamine, fentanyl or morphine; anginal preparations, e.g., diltiazem; antiallergics, e.g., cromoglycate, ketotifen or nedocromil; antiinfectives e.g., cephalosporins, penicillins, streptomycin, sulphonamides, tetracyclines and pentamidine; antihistamines, e.g., methapyrilene; anti-inflammatories, e.g., beclomethasone (eg. as the dipropionate), fluticasone (eg. as the propionate), flunisolide, budesonide, rofleponide, mometasone (e.g. as the furoate) or triamcinolone (e.g. as the acetonide); antitussives, e.g., noscapine; bronchodilators, e.g., albuterol (eg. as the sulphate), salmeterol (eg. as the xinafoate), ephedrine, adrenaline, fenoterol (eg. as the hydrobromide), formoterol (e.g. as the fumarate), isoprenaline, metaproterenol, phenylephrine, phenylpropanolamine, pirbuterol (eg. as the acetate), reproterol (eg. as the hydrochloride), rimiterol, terbutaline (eg. as the sulphate), isoetharine, tulobuterol or (-)-4-amino-3,5-dichloro-α-[[[6-[2-(2-pyridinyl)ethoxy] hexyl]methyl] benzenemethanol; diuretics, e.g., amiloride; anticholinergics, e.g., ipratropium (e.g. as the bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; and salts, esters and solvates of any of the above. Other examples include 4-hydroxy-7-[2-[[2-[[3-(2-phenylethoxy)propyl]sulfonyl]ethyl] amino]ethyl-2(3H)-benzothiazolone or butixicort and salts and solvates thereof. Another example of a pharmaceutical substance suitable for inhalation therapy is 6α, 9α-difluoro-11 β-hydroxy-16α-methyl-3-oxo-17α-propionyloxy-androsta-1,4-diene-17β-carbothioic acid S-(2-oxo-tetrahydro-furan-3-yl) ester or a solvate thereof (which compound is especially suitable for administration by the nasal route).
Other examples of pharmaceutical substances suitable for inhalation therapy which are of particular interest are:
(2R,3R,4S,5R)-2-[6-Amino-2-(1S -hydroxymethyl-2-phenyl-ethylamino)-purin-9-yl]-5-(2-ethyl-2H-tetrazol-5-yl)-tetrahydro-furan-3,4-diol or a salt thereof (eg. the maleate salt); and
(2S)-3-[4-({[4-(Aminocarbonyl)-1-piperidinyl]carbonyl)oxy)phenyl]-2-[((2S)-4-methyl-2-{[2-(2-methylphenoxy)acetyl]amino}pentanoyl)amino] propanoic acid or a salt thereof (eg. as free acid or potassium salt).

Examples of other pharmaceutical substances for which the process according to the invention is useful include compounds to be administered orally such as 2(S)-(2-benzoyl-phenylamino)-3-{4-[2-(5-methyl-2-phenyl-oxazol-4-yl)-ethoxy]-phenyl}-propionic acid, 2,6-diamino-3-(2,3,5-trichlorophenyl)pyrazine and naratriptan (eg. as hydrochloride) and other 5HT-1 agonists such as sumatriptan (eg. as succinate). Another compound of interest is (S)-[2-(1-iminoethylamino)ethyl]-L-homocysteine or a salt or racemate thereof (eg. preferably the 2- isomer).

Pharmaceutical substances as described above include asymmetric molecules which may exist as mixtures of optical isomers (e.g. as racemates) or as purified single enantiomers.

Pharmaceutical substances of particular interest include fluticasone, beclomethasone, salmeterol, salbutamol or an ester, salt or solvate thereof. The substance of most interest is salmeterol xinafoate (including the racemate or the purified r- or s- enantiomers). Fluticasone propionate is also of particular interest.

Examples of carrier substances include lactose.

The solvent and antisolvent liquids will be selected so as to be appropriate for the substance. Preferably, they are readily miscible in the proportions employed. Suitable combinations of solvent/antisolvent include acetone/water, ethanol/IPA, methanol/IPA, methanol/water and reciprocal pairs. Methanol/IPE is also a suitable pairing.

For generation of small particles by the process according to the invention, it is preferred that the difference between the dissolution properties of the solvent and anti-solvent be as great as possible. For reasons of industrial efficiency (particularly in order to reduce the throughput volumes of liquid) it is preferred to use concentrations of substance in solvent which are as high as possible. Nevertheless the solutions must be stable and not prone to crystallisation before discharge into the continuous flow cell. With this end in mind, it may be preferred to use the solution of the substance in the solvent at elevated temperature. It may also be preferable to cool the anti-solvent.

In order to prevent premature precipitation of the dissolved substance in the lines it will generally be desired to prime the apparatus by first pumping it with solvent. It may be preferred to prime the apparatus by pumping it with heated solvent, particularly when the dissolved substance is close to its solubility limit.

When the substance is fluticasone propionate we prefer the solvent to be acetone and the anti-solvent to be water.
When the substance is salmeterol xinafoate we prefer the solvent to be methanol or acetone (more preferably methanol) and the anti-solvent to be water.

When the substance is salbutamol sulphate we prefer the solvent to be water and the anti-solvent to be IMS.
When the substance is beclomethasone dipropionate we prefer the solvent to be IMS and the anti-solvent to be water.
When the substance is lactose we prefer the solvent to be water and the anti-solvent to be ethanol.
When the substance is budesonide, we prefer the solvent to be methanol and the anti-solvent to be water.
When the substance is formoterol fumarate or terbutaline sulphate we prefer the solvent to be methanol or acetone and the anti-solvent to be water.
When the substance is 2,6-diamino-3-(2,3,5-trichlorophenyl)pyrazine we prefer the solvent to be methanol and the anti-solvent to be water.
When the substance is 2(S)-(2-benzoyl-phenylamino)-3-{4-[2-(5-methyl-2-phenyl-oxazol-4-yl)-ethoxy]-phenyl}-propionic acid we prefer the solvent to be acetone and the anti-solvent to be water.
When the substance is naratriptan hydrochloride we prefer the solvent to be methanol and the antisolvent to be IPE.
When the substance is 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester we prefer the solvent to be acetone and the anti-solvent to be water.

We have found that the method according to the invention is suitable for producing populations of mixtures when the substance is a mixture of substances. When the substance is a mixture the method has particular advantages since it is capable of producing mixtures of crystalline particles of very high homogeneity without the need for any blending step. When the substance is a mixture the solvent and anti-solvent will have to be appropriate for all components of the mixture. Differential solubilities in the recrystalline mixture tend to result in the output proportions of the mixture differing from the initial proportions in solution in the solvent and so appropriate adjustment of the input proportions to achieve the desired output proportions may be necessary.

The method according to the invention is particularly suitable for producing mixtures of crystalline particles of salmeterol and fluticasone or salts and esters thereof e.g. salmeterol xinafoate and fluticasone propionate. The preferred solvent is acetone. The preferred anti-solvent is water. Recrystallisation from acetone using water as anti-solvent tends to cause an increase in the ratio of salmeterol xinafoate to fluticasone propionate relative to their proportion in solution in acetone. The method is also expected to be suitable for producing mixtures of crystalline particles of formoterol and budesonide or salts and esters thereof e.g. formoterol fumarate and budesonide.

As a further aspect of the invention we provide a population of particles obtainable by a process according to the invention.

Particles of pharmaceutical or carrier substances may be obtained which are suitable for use in a pharmaceutical composition for inhalation therapy, such as dry powder composition (whether containing pure drug, or drug mixed with a carrier such as lactose) or a pressurised liquid formulation (e.g. a formulation comprising a hydrofluoroalkane (HFA) propellant such as HFA134a or HFA227 or a mixture thereof).

Pressurised liquid formulations suitable for metered-dose inhalers will be retained in canisters, typically aluminium canisters (which may be plastics lined) which are provided with a metering valve of appropriate metering volume.

It will be appreciated that references to inhalation therapy also extend to administration of pharmaceutical compositions via the nasal route. Formulations suitable for nasal delivery include pressurised (e.g. HFA containing) formulations and non pressurised (e.g. aqueous) formulations which may be metered by the delivery device adapted for administration to the nose.

We also provide a pharmaceutical composition comprising a population of particles prepared according to the invention.

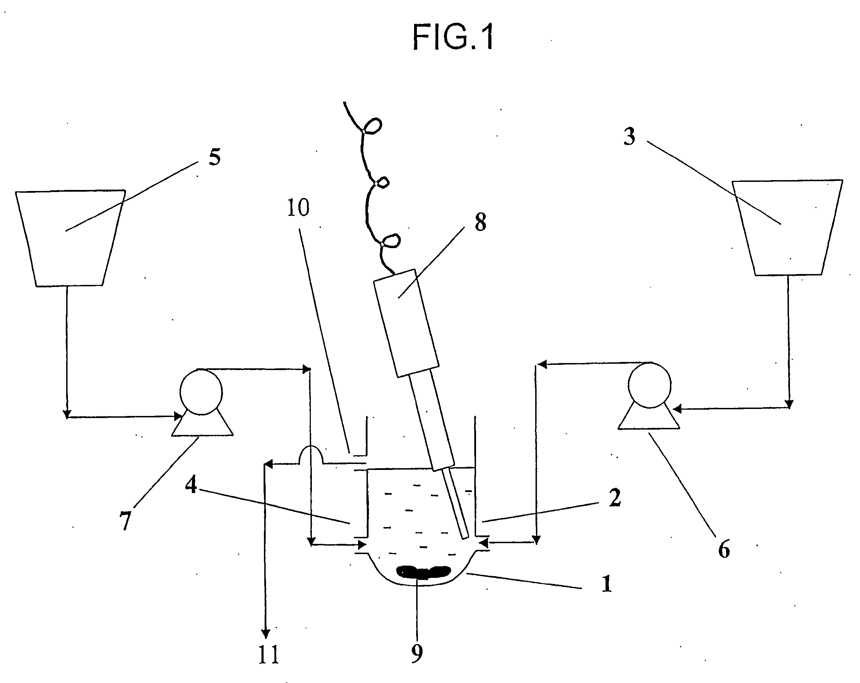

Apparatus suitable for use in the present invention is illustrated by reference to Figure 1 in which mixing chamber 1 is provided with first inlet port 2 connected to first reservoir 3 containing substance dissolved in solvent and second inlet port 4 connected to second reservoir 5 containing anti-solvent. Pumps 6 and 7 deliver liquid from reservoirs 3 and 5 to mixing chamber 1 at a controlled rate. An ultrasound probe 8 is located in the vicinity of, and just above, inlet port 2. When pumps 6 and 7 are in operation, liquids from reservoirs 3 and 5 are delivered to mixing chamber 1 and are mixed with the aid of magnetic stirrer 9. Liquid containing the particles of substance thus generated flows out of the mixing chamber via exit port 10. The solvent within this flowing suspension is then removed using a filter 11 according to the present invention.

### Brief description of the drawings.

Figure 1: Example apparatus according to the invention

The present invention may be illustrated by the following Example:

### Examples

### Example 1: Distributions of particles of crystalline fluticasone propionate

The drug substance (fluticasone propionate) (1wt) was dissolved in hot acetone (15vol) and then allowed to cool to ambient temperature (20°C). A flow cell was then charged with a 4:1 mixture of water and acetone respectively. Pump 1 (containing the fluticasone propionate in acetone) was set at a flow rate of 20ml/min. Pump 2 (containing water chilled to 3-5°C) was set at a flow rate of 80ml/min. A magnetic stirrer bar was placed in the flow cell. The tip of a sono-probe was positioned above the inlet of Pump 1 and set to deliver 70-75 watts of power. When the ultrasound probe, both pumps and the magnetic stirrer were turned on, rapid onset of crystallisation occurred.

The resultant crystalline suspension was then collected and simultaneously filtered on a filter funnel fitted with GF/C glass microfibre filter sandwiched between 2 Whatman No. 54 filter papers. The damp filter cake was then washed with water and then resuspended in further demineralised water to prepare a 10% w/w slurry. The slurry was then rapidly frozen by immersing the flask containing the slurry in a solid carbon dioxide cooling bath containing acetone, to give an even coating of ice containing particles of fluticasone propionate. The mixture was then freeze dried *in vacuo* for 14-18 hours to give a fine white powder containing particles of inhalable quality.

## Claims

1. A process for preparing crystalline particles of a substance which comprises mixing a flowing solution of the substance in a liquid solvent with a flowing liquid antisolvent for said substance in order to generate a suspension of crystalline particles in the solvent/anti-solvent the process further comprises the steps of
(a) filtering the suspension of crystalline particles in the solvent/anti-solvent mixture in order to remove the solvent/antisolvent mixture;
(b) washing the filtered particles with water;
(c) resuspending the filtered and washed particles in water;
(d) cooling the resultant suspension of filtered, washed and resuspended particles in water to freezing point; and
(e) collecting crystalline particles by removal of the water from the cooled suspension by freeze drying.

2. A process according to claim 1 wherein said mixing comprises mixing in a continuous flow cell in the presence of ultrasonic radiation.

3. A process according to claim 1 wherein said mixing comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused.

4. A process according to any one of claims 1 to 3 wherein the solvent is miscible with the anti-solvent.

5. A process according to any one of claims 1 to 4 wherein the suspension of crystalline particles in the solvent/anti-solvent mixture will be filtered using a filter which is suitable to retain crystalline particles of between 1 and 10µm.

6. A process according to claim 5 wherein the filter is suitable to retain crystalline particles of less than 5µm.

7. A process according to claim 6 wherein the filter is suitable to retain crystalline particles of less than 3µm.

8. A process according to any one of claims 1 to 7 wherein the anti-solvent is water.

9. A process according to any one of claims 1 to 8 wherein the suspension of crystalline particles obtained in step (d) are cooled to freezing point using a solid carbon dioxide cooling bath containing a suitable solvent eg. acetone, IMS or methanol.

10. A process according to any one of claims 1 to 9 wherein the process prepares particles of substances which are pharmaceutical or carrier substances suitable for inhalation therapy.

11. A process according to claim 10 wherein the substance is fluticasone, beclomethasone, salmeterol, salbutamol or an ester, salt or solvate thereof.

12. A process according to claim 10 wherein the substance is lactose.

13. A process according to claim 10 wherein the substance is 6α, 9α-difluoro-11β-hydroxy-16a-methyl-3-oxo-17α-propionyloxy-androsta-1,4-diene-17β-carbothioic acid S-(2-oxo-tetrahydro-furan-3-yl) ester.

14. A process according to claim 11 wherein the substance is fluticasone propionate.

15. A process according to claim 11 wherein the substance is salmeterol xinafoate.

16. A process according to any one of claims 1, 2 or 3 wherein the substance is a mixture.

17. A process according to claim 16 wherein the substance is a mixture of fluticasone propionate and salmeterol xinafoate.

18. A process according to any one of claims 1 to 9 wherein the process prepares particles of substances which may be administered orally.

19. A process according to claim 18 wherein the substance is 2(S)-(2-benzoyl-phenylamino)-3-{4-[2-(5-methyl-2-phenyl-oxazol-4-yl)-ethoxy]-phenyl}-propionic acid or 2,6-diamino-3-(2,3,5-trichlorophenyl)pyrazine.

20. A process according to claim 18 wherein the substance is naratriptan hydrochloride.

21. A population of particles obtainable by a process according to any one of claims 1 to 20.

22. A pharmaceutical composition comprising a population of particles according to claim 21.

## Patentansprüche

1. Verfahren zur Herstellung von kristallinen Partikeln aus einer Substanz, das das Vermischen einer fließenden Lösung der Substanz in einem flüssigen Lösungsmittel mit einem fließenden flüssigen Antilösungsmittel für die Substanz umfasst, um eine Suspension aus kristallinen Partikeln im Lösungsmittel/Antilösungsmittel zu erzeugen, wobei das Verfahren ferner die folgenden Schritte umfasst:
(a) Filtrieren der Suspension der kristallinen Partikel in der Lösungsmittel/Antilösungsmittel-Mischung zur Entfernung der Lösungsmittel/Antilösungsmittel-Mischung;
(b) Waschen der filtrierten Partikel mit Wasser;
(c) Resuspendieren der filtrierten und gewaschenen Partikel in Wasser;
(d) Abkühlen der resultierenden Suspension aus filtrierten, gewaschenen und resuspendierten Partikeln in Wasser auf den Gefrierpunkt; und
(e) Auffangen der kristallinen Partikel durch Entfernen des Wassers aus der abgekühlten Suspension durch Gefriertrocknung.

2. Verfahren gemäß Anspruch 1, worin das Vermischen das Vermischen in einer kontinuierlichen Durchflusszelle in Gegenwart von Ultraschall umfasst.

3. Verfahren gemäß Anspruch 1, worin das Vermischen das Zuführen eines Stroms von Lösung der Substanz in einem flüssigen Lösungsmittel und eines Stroms von flüssigem Antilösungsmittel für die Substanz tangential in eine zylindrische Mischkammer mit einer axialen Auslassöffnung umfasst, so dass die Ströme **dadurch** innig durch Bildung eines Wirbels vermischt werden und **dadurch** eine Ausfällung von kristallinen Partikeln aus der Substanz verursacht wird.

4. Verfahren gemäß einem der Ansprüche 1 bis 3, worin das Lösungsmittel mit dem Antilösungsmittel mischbar ist.

5. Verfahren gemäß einem der Ansprüche 1 bis 4, worin die Suspension aus kristallinen Partikeln in der Lösungsmittel/Antilösungsmittel-Mischung unter Verwendung eines Filters filtriert wird, der geeignet ist, um kristalline Partikel zwischen 1 und 10 µm zurückzuhalten.

6. Verfahren gemäß Anspruch 5, worin der Filter geeignet ist, um kristalline Partikel mit weniger als 5 µm zurückzuhalten.

7. Verfahren gemäß Anspruch 6, worin der Filter geeignet ist, um kristalline Partikel mit weniger als 3 µm zurückzuhalten.

8. Verfahren gemäß einem der Ansprüche 1 bis 7, worin das Antilösungsmittel Wasser ist.

9. Verfahren gemäß einem der Ansprüche 1 bis 8, worin die Suspension aus kristallinen Partikeln, die in Schritt (d) erhalten wird, auf den Gefrierpunkt unter Verwendung eines Kühlbades mit festem Kohlendioxid abgekühlt wird, das ein geeignetes Lösungsmittel enthält, z.B. Aceton, Brennspiritus oder Methanol.

10. Verfahren gemäß einem der Ansprüche 1 bis 9, worin das Verfahren Partikel aus Substanzen herstellt, die pharmazeutische oder Trägersubstanzen sind, die zur Inhalationstherapie geeignet sind.

11. Verfahren gemäß Anspruch 10, worin die Substanz Fluticason, Beclomethason, Salmeterol, Salbutamol oder ein Ester, Salz oder Solvat davon ist.

12. Verfahren gemäß Anspruch 10, worin die Substanz Lactose ist.

13. Verfahren gemäß Anspruch 10, worin die Substanz 6α,9α-Difluor-11β-hydroxy-16α-methyl-3-oxo-17α-propionyloxyandrosta-1,4-dien-17β-thiocarbonsäure-S-(2-oxotetrahydro-furan-3-yl)ester ist.

14. Verfahren gemäß Anspruch 11, worin die Substanz Fluticasonpropionat ist.

15. Verfahren gemäß Anspruch 11, worin die Substanz Salmeterolxinafoat ist.

16. Verfahren gemäß einem der Ansprüche 1, 2 oder 3, worin die Substanz eine Mischung ist.

17. Verfahren gemäß Anspruch 16, worin die Substanz eine Mischung aus Fluticasonpropionat und Salmeterolxinafoat ist.

18. Verfahren gemäß einem der Ansprüche 1 bis 9, worin das Verfahren Partikel aus Substanzen herstellt, die oral verabreicht werden können.

19. Verfahren gemäß Anspruch 18, worin die Substanz 2(S)-(2-Benzoyl-phenylamino)-3-{4-[2-(5-methyl-2-phenyl-oxazol-4-yl)-ethoxy]-phenyl}-propionsäure oder 2,6-Diamino-3-(2,3,5-trichlorphenyl)pyrazin ist.

20. Verfahren gemäß Anspruch 18, worin die Substanz Naratriptanhydrochlorid ist.

21. Population von Partikeln, die durch ein Verfahren gemäß einem der Ansprüche 1 bis 20 erhältlich sind.

22. Pharmazeutische Zusammensetzung, die eine Population von Partikeln gemäß Anspruch 21 umfasst.

## Revendications

1. Procédé pour la préparation de particules cristallines d'une substance qui comprend le mélangeage d'une solution fluide de la substance dans un solvant liquide avec un antisolvant liquide fluide pour ladite substance afin de générer une suspension de particules cristallines dans le solvant/antisolvant, le procédé comprenant, en outre, les étapes consistant à :
(a) filtrer la suspension de particules cristallines dans le mélange solvant/antisolvant afin d'éliminer le mélange solvant/antisolvant ;
(b) laver les particules filtrées à l'eau ;
(c) remettre en suspension les particules filtrées et lavées dans de l'eau ;
(d) refroidir la suspension obtenue de particules filtrées, lavées et remises en suspension dans de l'eau jusqu'au point de congélation ; et
(e) recueillir les particules cristallines en éliminant l'eau de la suspension refroidie par lyophilisation.

2. Procédé selon la revendication 1, dans lequel ledit mélangeage comprend un mélangeage dans une cellule à flux continu en présence d'une émission ultrasonique.

3. Procédé selon la revendication 1, dans lequel ledit mélangeage comprend l'admission d'un flux de la solution de la substance dans un solvant liquide et d'un flux d'antisolvant liquide pour ladite substance, de manière tangentielle dans une chambre de mélange cylindrique ayant un orifice de sortie axial de telle sorte que lesdits flux sont ainsi intimement mélangés par le biais de la formation d'un tourbillon et que la précipitation des particules cristallines de la substance est ainsi provoquée.

4. Procédé selon l'une quelconque des revendications 1 à 3, dans lequel le solvant est miscible avec l'antisolvant.

5. Procédé selon l'une quelconque des revendications 1 à 4, dans lequel la suspension de particules cristallines dans le mélange solvant/antisolvant est filtrée à l'aide d'un filtre qui est approprié pour retenir des particules cristallines allant de 1 à 10 *µ*m.

6. Procédé selon la revendication 5, dans lequel le filtre est approprié pour retenir des particules cristallines inférieures à 5 µm.

7. Procédé selon la revendication 6, dans lequel le filtre est approprié pour retenir des particules cristallines inférieures à 3 µm.

8. Procédé selon l'une quelconque des revendications 1 à 7, dans lequel l'antisolvant est de l'eau.

9. Procédé selon l'une quelconque des revendications 1 à 8, dans lequel la suspension de particules cristallines obtenues à l'étape (d) est refroidie jusqu'au point de congélation au moyen d'un bain de refroidissement de dioxyde de carbone solide contenant un solvant approprié, par exemple de l'acétone, de l'IMS ou du méthanol.

10. Procédé selon l'une quelconque des revendications 1 à 9, dans lequel le procédé prépare des particules de substances qui sont des substances pharmaceutiques ou des substances porteuses appropriées pour un traitement par inhalation.

11. Procédé selon la revendication 10, dans lequel la substance est la fluticasone, la béclométhasone, le salmétérol, le salbutamol ou un ester, un sel ou un solvant de celles-ci.

12. Procédé selon la revendication 10, dans lequel la substance est le lactose.

13. Procédé selon la revendication 10, dans lequel la substance est le S-(2-oxo-tétrahydro-furan-3-yl) ester d'acide 6α,9α-difluro-11β-hydroxy-16α-méthyl-3-oxo-17α-propionyloxy-androsta-1,4-diène-17 carbothioïque.

14. Procédé selon la revendication 11, dans lequel la substance est le propionate de fluticasone.

15. Procédé selon la revendication 11, dans lequel la substance est le xinafoate de salmétérol.

16. Procédé selon l'une quelconque des revendications 1, 2 ou 3, dans lequel la substance est un mélange.

17. Procédé selon la revendication 16, dans lequel la substance est un mélange de propionate de fluticasone et de xinafoate de salmétérol.

18. Procédé selon l'une quelconque des revendications 1 à 9, dans lequel le procédé prépare des particules de substances qui peuvent être administrées par voie orale.

19. Procédé selon la revendication 18, dans lequel la substance est l'acide 2(S)-(2-benzoyl-phénylamino)-3-{4-[2-(5-méthyl-2-phényl-oxazol-4-yl)-éthoxy]-phényl}-propionique ou la 2,6-diamino-3-(2,3,5-trichlorophényl)pyrazine.

20. Procédé selon la revendication 18, dans lequel la substance est le chlorhydrate de naratriptan.

21. Population de particules pouvant être obtenues par un procédé selon l'une quelconque des revendications 1 à 20.

22. Composition pharmaceutique comprenant une population de particules selon la revendication 21.
